(19) Europäisches Patentamt
European Patent Office
Office européen des brevets

(11) Numéro de publication: **0 218 506 B1**

(12) # FASCICULE DE BREVET EUROPEEN

(45) Date de publication du fascicule du brevet: **22.03.89**

(21) Numéro de dépôt: **86402014.4**

(22) Date de dépôt: **15.09.86**

(51) Int. Cl.⁴: **C07F 9/09**, C07F 9/58, C07H 19/06, C07H 19/16, C12N 11/02, C12N 11/14

(54) **Procédé d'insolubilisation d'enzymes par fixation sur des complexes alumine-phosphates organiques et enzymes insolubilisées obtenues par ce procédé, nouveaux supports d'enzymes et leur procédé de préparation et application desdits enzymes insolubilisées. 000**

(30) Priorité: **17.09.85 FR 8513740**

(43) Date de publication de la demande: **15.04.87 Bulletin 87/16**

(45) Mention de la délivrance du brevet: **22.03.89 Bulletin 89/12**

(84) Etats contractants désignés: **AT BE CH DE FR GB IT LI LU NL SE**

(56) Documents cités:
**CHEMICAL ABSTRACTS, vol. 81, no. 11, 16 septembre 1974, page 187, résumé no. 60071f, Columbus, Ohio, US; C.R. LOWE et al.: "Affinity chromatography on immobilized adenosine 5'-monophosphate. Some kinetic parameters involved in the binding of group-specific enzymes", & EUR. J. BIOCHEM. 1974, 42(1), 1-6 000**

(73) Titulaire: **INSTITUT NATIONAL DE LA SANTE ET DE LA RECHERCHE MEDICALE (INSERM), 101, rue de Tolbiac, F-75654 Paris Cédex 13(FR)**

(72) Inventeur: **Previero, Aldo, Mas de la Droleta Rue du Père Prévost, F-34100 Montpellier(FR)**
Inventeur: **Pugniere, Martine, 1 Grand Rue, Clapiers, F-34170 Castelnau Le Lez(FR)**
Inventeur: **Previero, Maria-Antonia, Mas de la Droleta Rue du Père Prévost, F-34100 Montpellier(FR)**

(74) Mandataire: **Orès, Bernard et al, Cabinet ORES 6, Avenue de Messine, F-75008 Paris(FR)**

**EP 0 218 506 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention est relative à un procédé d'insolubilisation d'enzymes par fixation sur des complexes alumine-phosphates organiques et aux enzymes insolubilisées obtenues par ce procédé; elle est également relative aux nouveaux supports d'enzymes, insolubles, et à leur procédé de préparation; elle est d'autre part relative aux applications des enzymes insolubilisées ainsi préparées.

La fixation d'enzymes à des supports insolubles constitue une étape clé en ce qui concerne la plupart des procédés de bioconversion dans le domaine de la technologie enzymatique.

Le choix du support insoluble et du type de fixation de l'enzyme visent à réaliser un compromis optimal entre plusieurs paramètres comme la stabilité chimique de la préparation, les propriétés mécaniques, la résistance à la biodégradation, le rapport surface/volume, la densité des molécules d'enzymes par unité de surface, le prix du support, la possibilité de régénération du support, etc...

Les supports insolubles généralements utilisés peuvent être classés en deux grands groupes:

a) les supports organiques qui comprennent les polyosides (cellulose et dérivés, dextranes et agarose, amidon, etc...), les protéines (collagène), les polymères synthétiques (polyaminoacides, résines polyacryliques, polyamides, etc...)
b) les supports inorganiques (verre poreux, oxydes métalliques, alumino-silicates, etc...)

Les supports organiques peuvent être chimiquement activés par différentes techniques, ce qui constitue l'un de leurs principaux avantages car ils permettent la fixation d'enzymes par différentes voies (inclusion, adsorption, liaison covalente,...).

Les supports inorganiques sont généralement plus stables (résistance à l'usure, aux agents chimiques et aux bactéries), mécaniquement, géométriquement et économiquement plus intéressants que les supports organiques.

L'activation chimique des supports inorganiques est par contre une opération plus difficile, plus onéreuse et moins efficace que dans le cas des supports organiques. C'est la raison pour laquelle la fixation d'enzymes à un support inorganique se fait de préférence par adsorption. Bien que toutes les enzymes soient adsorbables, ceci n'implique pas une application générale en raison des affinités très variables entre enzymes et supports.

La présente invention a pour but de pourvoir à de nouveaux supports insolubles qui allient la stabilité des supports inorganiques à la facilité d'activation des supports organiques, qui comportent des groupes fonctionnels réactifs permettant la fixation d'autres molécules simples ou complexes, lesdits supports insolubles ayant la faculté, après fixation d'enzymes par liaison covalente par l'intermédiaire de leurs groupes fonctionnels réactifs, de former des complexes enzymatiques insolubilisés qui présentent les avantages bien connus des enzymes immobilisées et qui, de plus, sont actifs non seulement dans l'eau et en milieu aqueux, mais le sont également dans des solvants organiques non miscibles à l'eau, et peuvent être utilisées pour catalyser des réactions menées aussi bien en continu qu'en discontinu.

La présente invention a pour objet un nouveau complexe solide qui est caractérisé en ce qu'il est formé par de l'alumine liée à au moins un groupe fonctionnel phosphate d'un composé bi- ou polyfonctionnel contenant au moins un groupe fonctionnel phosphate et au moins un groupe chimiquement réactif capable de former une liaison covalente avec des molécules de nature protéique, et notamment des enzymes.

Selon un mode de réalisation préféré du nouveau complexe conforme à la présente invention, le composé bi- ou polyfonctionnel lié à l'alumine est un phosphate organique qui répond à une des formules I ou II ci-après:

$$\underset{\mathrm{I}}{R\text{-}O\text{-}PO_3H_2} \qquad \underset{\mathrm{II}}{R\text{-}PO_3H_2}$$

dans lesquelles R représente un groupe organique aliphatique ou aromatique comprenant au moins un groupe chimiquement réactif tel, en particulier, qu'un groupe amine primaire, aldéhyde ou acide.

La présente invention a également pour objet un procédé de préparation du complexe alumine-phosphate organique conforme à l'invention, qui consiste à fixer un phosphate organique qui répond à la formule I ou II ci-dessus, sur de l'alumine, par mise en contact de l'alumine et d'un phosphate organique, pendant un temps suffisant, en milieu aqueux, sensiblement à la température ambiante, à un pH compris entre 2,0 et 8,5.

Selon un mode de mise en œuvre préféré du procédé de préparation du complexe alumine-phosphate organique conforme à l'invention, le milieu dans lequel est réalisée la complexation est un milieu à force ionique élevée.

Les inventeurs ont pu établir, en effet, que les phosphates organiques de formules I et II se lient très fortement à l'alumine, dans l'eau, à un pH compris entre 2 et 8,5, que les complexes ainsi obtenus sont stables dans des milieux à force ionique élevée, par exemple dans NaCl 2M, $(NH_4)_2SO_4$ 2M, et vis-à-vis de l'action des solvants organiques, miscibles ou non miscibles à l'eau, et que les phosphates organiques de formules I et II peuvent être séparés de l'alumine par déplacement par des solutions d'autres

phosphates organiques ou par des solutions d'acide phosphorique équilibrées à un pH compris entre 2 et 8,5.

La présente invention a également pour objet l'application du complexe alumine-phosphate organique conforme à l'invention en tant que support de fixation de molécules de nature protéique, et notamment d'enzymes.

Elle a en outre pour objet l'application du complexe alumine-phosphate organique conforme à l'invention en tant que moyen pour l'isolement et/ou le dosage de phosphates organiques contenus dans un milieu biologique, tels que mononucléotides, phosphoglucides, phospho-protéines, phospho-amino-acides, pyridoxal-phosphate, etc..., par liaison desdits phosphates organiques avec de l'alumine, en milieu aqueux, à un pH compris entre 2 et 8,5, et libération de ces phosphates organiques du complexe formé, par action d'une solution d'un autre phosphate organique ou d'acide phosphorique équilibrée à un pH compris entre 2,0 et 8,5.

La présente invention a également pour objet un procédé de préparation d'enzymes insolubilisées, qui consiste à former un corps complexe comprenant une enzyme liée par liaison covalente à un support solide de formule Ia ou IIa ci-après:

$$Al_2O_3\text{----}R\text{-}O\text{-}PO_3H_2 \qquad\qquad Al_2O_3\text{----}R\text{-}PO_3H_2$$

Ia IIa

par réaction des groupes chimiquement réactifs que comprend le groupe organique aliphatique ou aromatique R, avec les groupes réactifs des enzymes à fixer, éventuellement activés par des activateurs appropriés.

Selon un mode de mise en œuvre préféré de ce procédé, lorsque le complexe insoluble comprend des groupes réactifs carbonyliques tels que des groupes aldéhyde, introduits par le phosphate organique, ces groupes réactifs réagissent directement avec les fonctions amine des enzymes à fixer, en formant des bases de Schiff.

Selon une modalité avantageuse de ce mode de mise en œuvre, les bases de Schiff obtenues sont stabilisées par réduction à l'aide d'un agent réducteur approprié, notamment du cyanoborohydrure de sodium.

Selon un autre mode de mise en œuvre du procédé de préparation d'enzymes insolubilisées, lorsque le complexe insoluble comprend des groupes réactifs constitués par des groupes amines, ceux-ci sont activés par un activateur approprié tel qu'un réactif bifonctionnel des amines primaires pris dans le groupe qui comprend notamment le glutaraldéhyde, les diisocyanates, les diisothiocyanates, etc..., pour être rendus aptes à réagir avec les groupes amine des enzymes à fixer.

La présente invention englobe également les enzymes insolubilisées par association avec un complexe aluminephosphate organique, obtenues conformément à la présente invention.

La présente invention a en outre pour objet un procédé de catalyse de synthèses peptidiques à l'aide des enzymes insolubilisées définies dans ce qui précède, lesquelles synthèses peptidiques sont réalisées par synthèse de liaisons amidiques et peptidiques entre un composant carboxylique et un composant aminé dérivés d'acides aminés.

Selon un mode de mise en œuvre préféré de ce procédé, lesdites synthèses peptidiques sont réalisées à une température comprise entre la température ambiante et 45°C environ.

Selon un mode de mise en œuvre préféré du procédé de catalyse de synthèses peptidiques conforme à l'invention, le milieu réactionnel est exempt d'ions phosphate dont la présence pourrait provoquer la séparation du phosphate organique de l'alumine à laquelle il est lié et, par suite, la séparation de l'enzyme de son support insoluble.

Selon un autre mode de mise en œuvre préféré du procédé de catalyse de synthèses peptidiques conforme à l'invention, celui-ci catalyse la synthèse de liaisons amidiques et peptidiques entre un dérivé d'acide aminé présentant une fonction amine bloquée et une fonction carboxylique libre ou estérifiée et un dérivé d'acide aminé présentant une fonction amine libre et une fonction carboxylique bloquée, qui répondent respectivement aux formules III et IV ci-après:

$$Z\text{-}NH\text{-}\underset{\displaystyle R_x}{\underset{|}{C}}HCOOX \qquad (III)$$

où Z représente un groupe de protection de la fonction amine ou un segment peptidique
X représente un atome d'hydrogène ou un groupe alkyle,
$R_x$ représente la chaîne latérale d'un acide α-aminé;

$$H_2N-\underset{\displaystyle R_x}{\underset{|}{CH}}-COY \qquad (IV)$$

où Y représente un group alcoxy, alcoylamine, ou encore un segment peptidique et $R_x$ représente la chaîne latérale d'un acide α-aminé.

La présente invention vise plus particulièrement les nouveaux complexes alumine-phosphate organique et les nouvelles structures enzyme-complexe alumine-phosphate organique, conformes aux dispositions qui précèdent, ainsi que leurs applications, les procédés pour les préparer et les moyens mis en œuvre pour les préparer et pour les appliquer.

Outre les dispositions qui précèdent, l'invention comprend encore d'autres dispositions, qui ressortiront de la description qui va suivre.

L'invention sera mieux comprise à l'aide du complément de description qui va suivre, qui se réfère a des exemples de préparation des complexes alumine-phosphate organiques et des enzymes insolubilisées conformes à l'invention, ainsi qu'à des exemples d'applications de ces produits.

Il doit être bien entendu, toutefois que ces exemples sont donnés uniquement à titre d'illustration de l'objet de l'invention, dont ils ne constituent en aucune manière une limitation.

EXEMPLES

Exemples 1 à 7: Préparation de complexes alumine-phosphate-organique

A 2 ml d'une solution aqueuse $10^{-2}$ molaire d'un phosphate organique, on additionne 1 g d'alumine, utilisée soit sous forme de poudre, soit sous forme de microbilles poreuses, préalablement équilibrée avec un solvant approprié qui sera le même au cours des réactions dans lesquelles le complexe formé sera impliqué ultérieurement, et la suspension est maintenue sous agitation pendant 10 minutes à la température ambiante. Le phosphate présent dans le surnageant est analysé quantitativement par chromatographie liquide à haute pression, par spectrophométrie U.V. ou par colorimétrie, selon le cas, et le taux de fixation à l'alumine est déterminé par différence. Pour chaque phosphate l'expérience a été menée dans différentes conditions de pH, en présence de sels et en présence de phosphates inorganiques.

Le tableau 1 qui va suivre, indique pour chaque exemple, dans la 2ème colonne, le phosphate organique mis en œuvre, dans la 3ème colonne le pourcentage de fixation des différents phosphates sur l'alumine, et dans la 4ème colonne, le pourcentage de phosphate organique qui se fixe sur l'alumine en présence de phosphate inorganique.

**Tableau 1**

Pourcentage de fixation de phosphates organiques à l'alumine

| Exemples | Phosphate | de I à V* | Phosphate 0,3 M pH 3–8,5 |
|---|---|---|---|
| 1 | O-phosphosérine | 100 | 0 |
| 2 | O-phosphocolamine | 100 | 1 |
| 3 | Guanosine 5'-phosphate | 100 | 2 |
| 4 | Uridine 5'-phosphate | 100 | 1,5 |
| 5 | Adénosine 5'-phosphate | 100 | 1,5 |
| 6 | Acide cytidilique 3'-phosphate | 100 | 1 |
| 7 | Pyridoxal 5'-phosphate | 100 | 2 |

* I: $H_2O$ pH 7; II: NaCl 2 M pH 7; III: $CH_3COOH$ 0,5 M; IV: $CH_3COOH$ 0,5 M NaCl M; V: $(NH_4)_2SO_4$ M

Le caractère spécifique de l'inter-réaction alumine-phosphate est prouvé par des expériences comparatives avec des produits acides soit carboxyliques soit sulfoniques (Tableau 2).

**Tableau 2**

| | $CH_3COOH$ 0,5 M | $CH_3COOH$ 0,5 M, NaCl M |
|---|---|---|
| Sérine | 0 | 0 |
| Sérine O-sulfate | 40 | 2 |
| Colamine O-sulfate | 35 | 1 |

Exemples 8 et 9: Fixation d'une enzyme sur le complexe alumine-phosphocolamine préparé conformément à l'Exemple 2

1) A une solution de 60 mg de O-phosphocolamine dans 10 ml d'eau équilibrée à pH 7, on ajoute 3 g d'alumine. La suspension est maintenue à pH 7 sous légère agitation. Après 30 minutes, la phosphocolamine est totalement fixée sur l'alumine qui est récupérée par filtration ou centrifugation.

2) L'agrégat alumine-phosphocolamine est suspendu dans 10 ml d'eau et traité avec 4 ml d'une solution de glutaraldéhyde à 25% dans l'eau. La suspension est équilibrée à pH 7 dans l'eau et maintenue sous légère agitation pendant 30 minutes. L'alumine ainsi activée est à nouveau séparée et lavée à l'eau.

3) 50 mg d'enzyme sont dissous dans 10 ml d'eau dans lesquels on ajoute le complexe alumine-phosphocolamine activé comme décrit en 2). La suspension est maintenue à pH 7 sous légère agitation pendant 30 minutes. L'enzyme insolubilisée est récupérée par essorage et lavée à l'eau jusqu'à disparition complète de l'activité enzymatique dans l'eau de lavage.

Toutes les opérations décrites ont été menées à température ambiante.

On obtient 4,5 g d'enzyme immobilisée (soit 0,5 g d'eau par g. d'alumine de départ). L'activité de l'enzyme immobilisée est déterminée à l'aide de substrats spécifiques comme décrit dans le Tableau 3 qui va suivre.

Un tiers de la préparation est suspendu dans 20 ml de NaCl 1 M et maintenu sous agitation à pH 7 pendant 24 heures. La phase solide est récupérée par essorage, lavée à l'eau et l'activité enzymatique déterminée à nouveau.

**Tableau 3**

**Activité des préparations d'enzymes insolubilisées (u/g)**

| Exemple | Enzyme | Substrat | Préparation fraîche | Après traitement avec NaCl 1 M |
|---|---|---|---|---|
| 8 | Trypsine | Bz-ArgOEt | 62,5 | 63 |
| 9 | α-chymotrypsine | Ac.TyrOEt | 74 | 74 |

Exemple 10: Fixation d'une enzyme sur le complexe alumine-pyridoxal 5'-phosphate préparé conformément à l'exemple 7

1) A une solution de 20 mg de pyridoxal 5'-phosphate dans 10 ml d'eau, on ajoute 3 g d'alumine et la suspension est maintenue à pH 7 sous légère agitation. Après fixation complète du pyridoxal 5'-phosphate sur l'alumine, la phase solide, devenue jaune, est séparée par essorage et lavée à l'eau.

2) Une solution de 60 mg de trypsine dans 10 ml d'eau est ajoutée au complexe alumine-pyridoxal 5'-phosphate et la suspension maintenue sous agitation à pH 7 et à température ambiante. Après 30 minutes, 30 mg de cyanoborohydrure de sodium sont ajoutés à la suspension qui est maintenue sous agitation pendant 30 minutes supplémentaires à pH 7. Pendant ce temps la couleur de la suspension vire du jaune au blanc. La trypsine immobilisée est récupérée par essorage et lavée abondamment à l'eau. On obtient 4,5 g de trypsine immobilisée. Après avoir déterminé l'activité enzymatique, une partie de la préparation est mise en suspension dans un excès de NaCl 1M dans l'eau pendant 24 h sous légère agitation. On détermine l'activité enzymatique sur la phase solide séparée par filtration et lavée à l'eau.

**Tableau 4**

**Activité des préparations d'enzymes insolubilisées (u/g)**

| Exemple | Enzyme | Substrat | Préparation fraîche | Après traitement avec NaCl 1 M |
|---|---|---|---|---|
| 10 | Trypsine | Bz-ArgOEt | 73 | 73 |

Exemples 11 à 17: Synthèse peptidique par catalyse enzymatique

Dans une expérience type, 1 g de chymotrypsine immobilisée comme décrit dans l'exemple 9, est suspendu dans 2 ml de solvant organique (voir Tableau 5) contenant $10^{-4}$ moles d'ester méthylique d'acétyl L-tryptophane comme composant carboxylique et $10^{-3}$ moles d'ester méthylique de la glycine comme composant aminé. La suspension est maintenue sous légère agitation pendant 2 h à 20°C. L'acétyl-L-tryptophanyl-glycine ester formé est analysé quantitativement par chromatographie liquide haute pression à

l'aide d'un étalon synthétisé par voie chimique. De manière identique d'autres composants carboxyliques et aminés ont été condensés par catalyse chymotrypsique et les résultats sont donnés dans le Tableau 5.

Tableau 5

| Exemple n° | Composé carboxylique | Composé aminé | Acétate d'éthyle | 1,2-dichloro éthane |
|---|---|---|---|---|
| 11 | Ac.L.TrpOMe | GlyOMe | 52 | 49 |
| 12 | Ac.L.TrpOMe | benzylamine | 60 | 58 |
| 13 | Ac.L.PheOMe | GlyOMe | 60 | 60 |
| 14 | Ac.L.PheOMe | benzylamine | 65 | 70 |
| 15 | Z.L.Ala.L.TrpOMe | GlyOMe | 43 | 38 |
| 16 | Z.L.Ala.L.TrpOMe | benzylamine | 45 | 46 |
| 17 | For.l.TrpOMe | L.Leu.L.Asp.L.Phe$NH_2$ | 58 | 55* |

* Rendement déterminé par voie biologique. Le produit For.TrpLeuAspPhe$NH_2$ (formyl-tétragastrine) est un agent de sécrétion gastrique acide.

Exemple 18: Utilisation répétitive d'α-chymotrypsine en phase organique

1 g d'α-chymotrypsine immobilisée comme décrit dans l'exemple 9 sont suspendus dans 2 ml de 1,2 dichloroéthane contenant $10^{-4}$ moles d'Ac.L.TrpOMe et $10^{-3}$ moles de benzylamine. La suspension est placée dans un réacteur en verre muni d'une jaquette à circulation d'eau et fermé en haut et en bas par deux bouchons munis de filtres et de robinets. Après 2 h de réaction à 20°C, le solvant est séparé par filtration à l'aide d'une pression d'azote. L'acétyl-L-Trp benzylamide formé est analysé quantitativement sur un aliquot, par chromatographie liquide haute pression. L'enzyme immobilisée est lavée au dichloroéthane contenant 5% d'eau et utilisée pour un deuxième cycle dans les mêmes conditions que pour le premier cycle. Les rendements de synthèse à chaque cycle sont donnés dans le Tableau 6 ci-après.

Tableau 6

| Exemple | Cycle n° | 1 | 2 | 3 | 4 | 5 | 6 | 7 | 8 |
|---|---|---|---|---|---|---|---|---|---|
| 18 | synthèse % | 58 | 59 | 58 | 58 | 60 | 62 | 65 | 65 |

La légère augmentation du rendement peut provenir de la récupération du produit adsorbé au cours des premiers cycles.

Exemples 19 et 20: Thermolysine insolubilisée en synthèse peptidique

1) 3 g d'alumine sont complexés avec la O-phosphocolamine et activés par le glutaraldéhyde comme décrit précédemment (Ex. 8 et 9) et ajoutés à 10 ml d'une solution $3 \cdot 10^{-2}$ M d'acétate de calcium contenant 50 mg de thermolysine. La suspension est maintenue 30 minutes sous légère agitation à température ambiante et à pH 7. L'analyse spectrale du surnageant montre que la totalité de l'enzyme a été fixée. La thermolysine ainsi immobilisée est lavée avec une solution $3 \cdot 10^{-2}$ M d'acétate de calcium et récupérée par essorage. On obtient 4,5 g de thermolysine immobilisée.

2) $4 \cdot 10^{-4}$ moles d'acide N-carbobenzoxy L-aspartique (Z.L-Asp) et $0,8 \cdot 10^{-3}$ moles d'ester méthylique de la L-phénylalanine (L-PheOMe) sont dissous dans 4 ml d'eau à pH 8 et à cette solution on ajoute encore 1 g de thermolysine immobilisée. La suspension est maintenue sous légère agitation à pH 8 et à 40°C pendant 10 h. Un produit solide précipite en se mélangeant à l'enzyme immobilisée. Après acidification à pH 5 avec de l'acide acétique, 4 ml de n-butanol sont ajoutés à la suspension. Le produit solide formé au cours de la réaction passe en solution. L'enzyme immobilisée est séparée par filtration, rééquilibrée dans une solution d'acétate de calcium $3 \cdot 10^{-2}$ M à pH 8 et utilisée pour les cycles successifs. Les deux phases liquides sont séparées et un aliquot de la phase organique analysé par chromatographie liquide haute pression. Le rendement en Z.L-Asp-L-PheOMe est déterminé à l'aide d'un étalon préparé chimiquement.

La condensation entre Z.L-Trp et L-LeuOMe a été conduite de manière analogue.

Tableau 7

| Exemple | Composé carboxylique | Composé aminé | Synthèse % I cycle | Synthèse % II cycle |
|---|---|---|---|---|
| 19 | Z.Asp | L.PheOMe | 60 | 58 |
| 20 | Z.L.Trp | L.LeuOMe | 100 | 100 |

Exemples 21 et 22: <u>Séparation chromatographique de phosphates organiques</u>

1) Une colonne en verre ($\ell$ = 3 cm, ∅ = 0,7 cm) est remplie de poudre d'alumine et équilibrée avec une solution 0,2M de NaCl dans l'eau ajustée à pH 3 avec HCl.

2) Un mélange d'Adénosine ($10^{-5}$ moles) et Adénosine-5'-phosphate ($10^{-5}$ moles) est introduit en haut de la colonne qui est éluée avec une solution 0,2M de NaCl à pH 3. Le débit est de 18 ml/heure et l'effluent est collecté en fraction de 0,6 ml.

3) Après un volume de 18 ml, on ajoute à l'éluant de l'acide phosphorique pour avoir une concentration de 2%, et on équilibre à pH 3 avec $NH_4OH$. On continue l'élution et le fractionnement.

L'analyse des différentes fractions a donné les résultats présentés dans le Tableau 8 ci-dessous.

Un mélange de Sérine, Sérine-0-sulfate et Sérine-0-phosphate a été séparé chromatographiquement de manière analogue. Les résultats sont réunis dans le Tableau 9 ci-après.

**Tableau 8**

**Isolement chromatographique de l'Adénosine-5'-phosphate Exemple 21**

| Composé | Effluent | N° Fraction | Rendement de récupération |
|---|---|---|---|
| Adénosine | NaCl 0,2 M pH 3 | 2–6 | 95% |
| Adénosine-5'-phosphate | NaCl 0,2 M $H_3PO_4$ 2% pH 3 | 34–40 | 92% |

**Tableau 9**

**Isolement chromatographique de la Sérine-0-phosphate Exemple 22**

| Composé | Effluent | N° Fraction | Rendement de récupération |
|---|---|---|---|
| Sérine | NaCl 0,2 M pH 3 | 1–3 | 94% |
| Sérine-0-Sulfate | NaCl 0,2 M pH 3 | 4–8 | 96% |
| Sérine-0-Phosphate | NaCl 0,2 M $H_3PO_4$ 2% pH 3 | 35–39 | 91% |

Les enzymes insolubilisées conformes à la présente invention peuvent être utilisées en tant que catalyseurs de synthèse peptidiques aussi bien dans des procédés en discontinu (dans des réacteurs du type batch ou sur colonnes) que dans des procédés en continu.

Du fait qu'elles sont disponibles sous forme de poudres, elles sont aisément manipulables, leurs conditions de mise en œuvre sont simples et elles procurent des rendements économiquement intéressants pour la production de composés, comme les peptides, qui présentent un intérêt croissant dans le domaine des médicaments, et qui sont, dans certains cas, plus faciles à préparer par catalyse enzymatique que par voie chimique, et ceci sans le risque d'une racémisation préjudiciable à leur activité.

Ainsi que cela ressort de ce qui précède, l'invention ne se limite nullement à ceux de ses modes de mise en œuvre, de réalisation et d'application qui viennent d'être décrits de façon plus explicite; elle en embrasse au contraire toutes les variantes qui peuvent venir à l'esprit du technicien en la matière, sans s'écarter du cadre, ni de la portée de la présente invention.

**Revendications pour les Etats contractants BE, CH, DE, FR, GB, IT, LI, LU, NL, SE**

1. Nouveau complexe solide qui est caractérisé en ce qu'il est formé par de l'alumine liée à au moins un groupe fonctionnel phosphate d'un composé bi- ou polyfonctionnel contenant au moins un groupe fonctionnel phosphate et au moins un groupe chimiquement réactif capable de former une liaison covalente avec des molécules de nature protéique, et notamment des enzymes.

2. Complexe selon la Revendication 1,caractérisé en ce que le composé bi- ou polyfonctionnel lié à l'alumine est un phosphate organique qui répond à l'une des formules I ou II ci-après:

$$R - O - PO_3H_2 \qquad \text{I}$$

$$R - PO_3H_2 \qquad \text{II}$$

dans lesquelles R représente un groupe organique aliphatique ou aromatique comprenant au moins un groupe chimiquement réactif tel, en particulier, qu'un groupe amine primaire, aldéhyde ou acide.

3. Procédé de préparation du complexe selon l'une quelconque des Revendications 1 ou 2, caractérisé en ce qu'il consiste à fixer un phosphate organique qui répond à la formule I ou II selon la Revendication 2, sur de l'alumine, par mise en contact de l'alumine et d'un phosphate organique, pendant un temps suffisant, en milieu aqueux, sensiblement à la température ambiante, à un pH compris entre 2,0 et 8,5.

4. Procédé selon la Revendication 3, caractérisé en ce que le milieu dans lequel est réalisée la complexation est un milieu à force ionique élevée.

5. Application du complexe selon l'une quelconque des Revendications 1 ou 2 en tant que support de fixation de molécules de nature protéique, et notamment d'enzymes.

6. Application du complexe selon l'une quelconque des Revendications 1 ou 2 en tant que moyen pour l'isolement et/ou le dosage de phosphates organiques contenus dans un milieu biologique tels que mononucléotides, phosphoglucides, phospho-protéines, phospho-amino-acides, pyridoxal-phosphate, etc... par liaison desdits phosphates organiques avec de l'alumine, en milieu aqueux, à un pH compris entre 2 et 8,5, et libération de ces phosphates organiques du complexe formé, par action d'une solution de phosphate inorganique ou d'acide phosphorique équilibrée à un pH compris entre 2,0 et 8,5.

7. Procédé de préparation d'enzymes insolubilisées, caractérisé en ce qu'il consiste à former un corps complexe comprenant une enzyme liée par liaison covalente à un support solide de formule Ia ou IIa ci-après:

$$Al_2O_3 \text{ ---- } R\text{-}O\text{-}PO_3H_2 \qquad \text{Ia}$$

$$Al_2O_3 \text{ ---- } R\text{-}PO_3H_2 \qquad \text{IIa}$$

par réaction des groupes chimiquement réactifs qui comprend le groupe organique aliphatique ou aromatique R, avec les groupes réactifs des enzymes à fixer, éventuellement activés par des activateurs appropriés.

8. Procédé selon la Revendication 7, caractérisé en ce que lorsque le complexe insoluble comprend des groupes réactifs carbonyliques tels que des groupes aldéhyde, introduits par le phosphate organique, ces groupes réactifs réagissent directement avec les fonctions amine des enzymes à fixer, en formant des bases de Schiff.

9. Procédé selon la Revendication 8, caractérisé en ce que les bases de Schiff obtenues sont stabilisées par réduction à l'aide d'un agent réducteur approprié, notamment du cyanoborohydrure de sodium.

10. Procédé selon la Revendication 7, caractérisé en ce que lorsque le complexe insoluble comprend des groupes réactifs constitués par des groupes amine, ceux-ci sont activés par un activateur approprié tel qu'un réactif bifonctionnel des amines primaires pris dans le groupe qui comprend notamment le glutaraldéhyde, les diisocyanates, les diisothiocyanates, etc.., pour être rendus aptes à réagir avec les groupes amine des enzymes à fixer.

11. A titre de produits industriels nouveaux, des enzymes insolubilisées par association avec un complexe alumine-phosphate organique, obtenues en mettant en œuvre le procédé selon l'une quelconque des Revendications 7 à 10.

12. Procédé de catalyse de synthèses peptidiques caractérisé par la mise en œuvre en tant que catalyseur enzymatique, d'au moins une enzyme insolubilisée conforme à la Revendication 11.

13. Procédé selon la Revendication 12, caractérisé en ce que lesdites synthèses peptidiques sont réalisées à une température comprise entre la température ambiante et 45°C environ.

14. Procédé selon l'une quelconque des Revendications 12 ou 13, caractérisé en ce que le milieu réactionnel est exempt d'ions phosphate dont la présence pourrait provoquer la séparation de l'enzyme et de son support.

15. Procédé selon l'une quelconque des Revendications 12 à 14, caractérisé en ce que les enzymes insolubilisées catalysent la synthèse de liaisons amidiques et peptidiques entre un dérivé d'acide aminé présentant une fonction amine bloquée et une fonction carboxylique libre ou estérifiée et un dérivé d'acide aminé présentant une fonction amine libre et une fonction carboxylique bloquée, qui répondent respectivement aux formules III et IV ci-après.

$$Z - NH-\underset{\displaystyle R_x}{\underset{|}{C}}HCOOX \qquad (III)$$

où Z représente un groupe de protection de la fonction amine ou un segment peptidique.
X représente un atome d'hydrogène ou un groupe alkyle
$R_x$ représente la chaîne latérale d'un acide α-aminé;

$$H_2N - \underset{\displaystyle R_x}{\underset{|}{C}}H - COY \qquad (IV)$$

où Y représente un groupe alcoxy ou alcoylamine, ou encore un segment peptidique et
$R_x$ représente la chaîne latérale d'un acide α-aminé.

**Revendications pour l'Etat contractant AT**

1. Procédé de préparation du complexe solide formé par de l'alumine liée à au moins un groupe fonctionnel phosphate d'un composé bi- ou polyfonctionnel contenant au moins un groupe fonctionnel phosphate et au moins un groupe chimiquement réactif capable de former une liaison covalente avec des molécules de nature protéique, et notamment des enzymes, caractérisé en ce qu'il consiste à fixer un phosphate organique qui répond à la formule I ou II ci-après:

$$R - O - PO_3H_2 \quad (I) \qquad\qquad R - PO_3H_2 \quad (II)$$

dans lesquelles:
R représente un groupe organique aliphatique ou aromatique comprenant au moins un groupe chimiquement réactif tel, en particulier, qu'un groupe amine primaire, aldéhyde ou acide,
sur de l'alumine, par mise en contact de l'alumine et d'un phosphate organique, pendant un temps suffisant, en milieu aqueux, sensiblement à la température ambiante, à un pH compris entre 2,0 et 8,5.

2. Procédé selon la Revendication 1, caractérisé en ce que le milieu dans lequel est réalisée la complexation est un milieu à force ionique élevée.

3. Procédé de préparation d'enzymes insolubilisées, caractérisé en ce qu'il consiste à former un corps complexe comprenant une enzyme liée par liaison covalente à un support solide de formule Ia ou IIa ci-après:

$$Al_2O_3 \text{ ---- } R-O-PO_3H_2 \quad (Ia) \qquad\qquad Al_2O_3 \text{ ---- } R-PO_3H_2 \quad (IIa)$$

par réaction des groupes chimiquement réactifs qui comprend le groupe organique aliphatique ou aromatique R, avec les groupes réactifs des enzymes à fixer, éventuellement activés par des activateurs appropriés.

4. Procédé selon la Revendication 3, caractérisé en ce que lorsque le complexe insoluble comprend des groupes réactifs carbonyliques tels que des groupes aldéhyde, introduits par le phosphate organique, ces groupes réactifs réagissent directement avec les fonctions amine des enzymes à fixer, en formant des bases de Schiff.

5. Procédé selon la Revendication 4, caractérisé en ce que les bases de Schiff obtenues sont stabilisées par réduction à l'aide d'un agent réducteur approprié, notamment du cyanoborohydrure de sodium.

6. Procédé selon la Revendication 3, caractérisé en ce que lorsque le complexe insoluble comprend des groupes réactifs constitués par des groupes amine, ceux-ci sont activés par un activateur approprié tel qu'un réactif bifonctionnel des amines primaires pris dans le groupe qui comprend notamment le glutaraldéhyde, les diisocyanates, les diisothiocyanates, etc.., pour être rendus aptes à réagir avec les

groupes amines des enzymes à fixer.

7. Procédé de catalyse de synthèse peptidique caractérisé par la mise en œuvre en tant que catalyseur enzymatique, d'au moins une enzyme insolubilisée par association avec un complexe alumine-phosphate organique, obtenue en mettant en œuvre le procédé selon l'une quelconque des Revendications 3 à 6.

8. Procédé selon la Revendication 7, caractérisé en ce que lesdites synthèses peptidiques sont réalisées à une température comprise entre la température ambiante et 45°C environ.

9. Procédé selon l'une quelconque des Revendications 7 ou 8, caractérisé en ce que le milieu réactionnel est exempt d'ions phosphate dont la présence pourrait provoquer la séparation de l'enzyme et de son support.

10. Procédé selon l'une quelconque des Revendications 7 à 9 , caractérisé en ce que les enzymes insolubilisées catalysent la synthèse de liaisons amidiques et peptidiques entre un dérivé d'acide aminé présentant une fonction amine bloquée et une fonction carboxylique libre ou estérifiée et un dérivé d'acide aminé présentant une fonction amine libre et une fonction carboxylique bloquée, qui répondent respectivement aux formules III et IV ci-après:

$$Z - NH-\underset{\displaystyle R_x}{\underset{|}{CH}}COOX \qquad (III)$$

où Z représente un groupe de protection de la fonction amine ou un segment peptidique.
X représente un atome d'hydrogène ou un groupe alkyle
$R_x$ représente la chaîne latérale d'un acide α-aminé;

$$H_2N - \underset{\displaystyle R_x}{\underset{|}{CH}} - COY \qquad (IV)$$

où Y représente un groupe alcoxy ou alcoylamine, ou encore un segment peptidique et
$R_x$ représente la chaîne latérale d'un acide α-aminé.

11. Application du complexe selon la Revendication 1, en tant que support de fixation de molécules de nature protéique, et notamment d'enzymes.

12. Application du complexe selon la Revendication 1, en tant que moyen pour l'isolement et/ou le dosage de phosphates organiques contenus dans un milieu biologique tels que mononucléotides, phosphoglucides, phospho-protéines, phospho-amino-acides, pyridoxal-phosphate, etc... par liaison desdits phosphates organiques avec de l'alumine, en milieu aqueux, à un pH compris entre 2 et 8,5, et libération de ces phosphates organiques du complexe formé, par action d'une solution de phosphate inorganique ou d'acide phosphorique équilibrée à un pH compris entre 2,0 et 8,5.

**Patentansprüche für die Vertragsstaaten BE, CH, DE, FR, GB, IT, LI, LU, NL, SE**

1. Neuer fester Komplex, dadurch gekennzeichnet, daß er gebildet ist aus Aluminiumoxid, gebunden an mindestens eine funktionelle Phosphatgruppe einer bi- oder polyfunktionellen Verbindung, die mindestens eine funktionelle Phosphatgruppe und mindestens eine chemisch reaktionsfähige Gruppe enthält, welche in der Lage ist, eine kovalente Bindung mit Protein-Molekülen, vor allem mit Enzymen, auszubilden.

2. Komplex nach Anspruch 1, dadurch gekennzeichnet, daß die an das Aluminiumoxid gebundene bi- oder polyfunktionelle Verbindung ein organisches Phosphat ist, das einer der folgenden Formeln I oder II entspricht,

$$R - O - PO_3H_2 \qquad\qquad R - PO_3H_2$$

(I) (II)

in denen R eine aliphatische oder aromatische organische Gruppe bedeutet, die mindestens eine chemisch reaktionsfähige Gruppe, wie insbesondere eine primäre Aminogruppe, eine Aldehydgruppe oder eine Säuregruppe, umfaßt.

3. Verfahren zur Herstellung des Komplexes nach einem der Ansprüche 1 oder 2, dadurch gekennzeichnet, daß man ein organisches Phosphat, das der Formel I oder II nach Anspruch 2 entspricht, auf Tonerde fixiert durch Inberührungbringen der Tonerde und eines organischen Phosphates während einer ausreichenden Zeit in wäßrigem Medium bei praktisch Raumtemperatur und bei einem pH-Wert von 2,0 bis 8,5.

4. Verfahren nach Anspruch 3, dadurch gekennzeichnet, daß das Medium, in welchem die Komplexbildung erfolgt, ein Medium hoher Ionenstärke ist.

5. Anwendung des Komplexes nach einem der Ansprüche 1 oder 2 als Träger für die Fixierung von Protein-Molekülen, insbesondere von Enzymen.

6. Anwendung des Komplexes nach einem der Ansprüche 1 oder 2 als Mittel für die Isolierung und/oder Bestimmung von organischen Phosphaten, die in einem biologischen Medium enthalten sind, wie Mononucleotide, Phosphoglucide, Phospho-Proteine, Phospho-Aminosäuren, Pyridoxal-phosphat usw. ..., durch Bindung dieser organischen Phosphate an Aluminiumoxid in wäßrigem Medium bei einem pH-Wert zwischen 2 und 8,5 und Freisetzung dieser organischen Phosphate aus dem entstandenen Komplex durch Einwirkung einer Lösung eines anorganischen Phosphates oder von Phosphorsäure, equilibriert auf einen pH-Wert im Bereich zwischen 2,0 und 8,5.

7. Verfahren zur Herstellung von unlöslich gemachten Enzymen, dadurch gekennzeichnet, daß man einen komplexen Körper bildet, umfassend ein Enzym, das kovalent an einen festen Träger der folgenden Formel Ia oder IIa

$$Al_2O_3 \text{ ---- } R\text{-}O\text{-}PO_3H_2 \quad (Ia) \qquad\qquad Al_2O_3 \text{ ---- } R\text{-}PO_3H_2 \quad (IIa)$$

gebunden ist, mittels Reaktion der im organischen aliphatischen oder aromatischen Rest R enthaltenen chemisch reaktionsfähigen Gruppen mit den reaktionsfähigen Gruppen der Enzyme, die fixiert werden sollen, gegebenenfalls aktiviert durch geeignete Aktivatoren.

8. Verfahren nach Anspruch 7, dadurch gekennzeichnet, daß, wenn der unlösliche Komplex reaktionsfähige Carbonylgruppen, wie Aldehydgruppen, umfaßt, die durch das organische Phosphat eingebracht worden sind, diese reaktionsfähigen Gruppen direkt mit den Aminogruppen der Enzyme, die fixiert werden sollen, unter Bildung von Schiff'schen Basen reagieren.

9. Verfahren nach Anspruch 8, dadurch gekennzeichnet, daß die erhaltenen Schiff'schen Basen durch Reduktion mit einem geeigneten Reduktionsmittel, insbesondere Natriumcyanborhydrid, stabilisiert werden.

10. Verfahren nach Anspruch 7, dadurch gekennzeichnet, daß, wenn der unlösliche Komplex reaktionsfähige Aminogruppen enthält, diese durch einen geeigneten Aktivator, wie einem bifunktionellen Reaktionspartner der primären Amine aus der Gruppe umfassend vor allem Glutaraldehyd, Diisocyanate, Diisothiocyanate usw. . . ., aktiviert werden, um dazu fähig gemacht zu werden, mit den Aminogruppen der Enzyme, die fixiert werden sollen, zu reagieren.

11. Als neue gewerbliche Produkte durch Kombination mit einem Aluminiumoxid-organischen Phosphat unlöslich gemachte Enzyme, erhalten durch Ausführen des Verfahrens nach einem der Ansprüche 7 bis 10.

12. Verfahren zum Katalysieren von Peptid-Synthesen, dadurch gekennzeichnet, daß man als enzymatischen Katalysator mindestens ein unlöslich gemachtes Enzym nach Anspruch 11 einsetzt.

13. Verfahren nach Anspruch 12, dadurch gekennzeichnet, daß die Peptid-Synthesen bei einer Temperatur zwischen Raumtemperatur und etwa 45°C ausgeführt werden.

14. Verfahren nach einem der Ansprüche 12 oder 13, dadurch gekennzeichnet, daß das Reaktionsmedium frei ist von Phosphationen, deren Anwesenheit die Trennung des Enzyms von seinem Träger hervorrufen könnten.

15. Verfahren nach einem der Ansprüche 12 bis 14, dadurch gekennzeichnet, daß die unlöslich gemachten Enzyme die Synthese von Amidbindungen und Peptidbindungen katalysieren zwischen einem Aminosäurederivat, das eine blockierte Aminogruppe und eine freie oder veresterte Carboxylgruppe aufweist, und einem Aminosäurederivat, das eine freie Aminogruppe und eine blockierte Carboxylgruppe aufweist, die jeweils den nachfolgenden Formeln III und IV entsprechen

$$Z - NH-\underset{\displaystyle R_x}{\underset{|}{C}}HCOOX \qquad (III)$$

in der Z eine Amino-Schutzgruppe oder ein Peptid-Segment darstellt, X ein Wasserstoffatom oder eine Alkylgruppe bedeutet und $R_x$ die Seitenkette einer α-Aminosäure ist;

$$H_2N - \underset{\underset{R_x}{|}}{CH} - COY \qquad (IV)$$

in der Y eine Alkoxy- oder Alkoylaminogruppe oder ein Peptid-Segment bedeutet und $R_x$ die Seitenkette einer α-Aminosäure ist.

**Patentansprüche für den Vertragsstaat AT**

1. Verfahren zur Herstellung des festen Komplexes, gebildet aus Aluminiumoxid, gebunden an mindestens eine funktionelle Phosphatgruppe einer bi- oder polyfunktionellen Verbindung, die mindestens eine funktionelle Phosphatgruppe und mindestens eine chemisch reaktionsfähige Gruppe enthält, die eine kovalente Bindung mit Protein-Molekülen, insbesondere mit Enzymen, ausbilden kann, dadurch gekennzeichnet, daß man ein organisches Phosphat, das der folgenden Formel I oder II entspricht

$$R - O - PO_3H_2 \quad (I) \qquad\qquad R - PO_3H_2 \quad (II)$$

in denen R einen organischen aliphatischen oder aromatischen Rest bedeutet, der mindestens eine chemisch reaktionsfähige Gruppe, wie insbesondere eine primäre Aminogruppe, Aldehydgruppe oder Säuregruppe, umfaßt, auf Aluminiumoxid fixiert durch Inberührungbringen des Aluminiumoxids mit einem organischen Phosphat während einer ausreichenden Zeitspanne in wäßrigem Medium, praktisch bei Raumtemperatur, bei einem pH-Wert zwischen 2,0 und 8,5.

2. Verfahren nach Anspruch 1, dadurch gekennzeichnet, daß das Medium, in welchem die Komplexbildung erfolgt, ein Medium hoher Ionenstärke ist.

3. Verfahren zur Herstellung von unlöslich gemachten Enzymen, dadurch gekennzeichnet, daß man einen komplexen Körper bildet, umfassend ein Enzym, das kovalent an einen festen Träger der folgenden Formel Ia oder IIa

$$Al_2O_3 \text{ ---- } R\text{-}O\text{-}PO_3H_2 \quad (Ia) \qquad\qquad Al_2O_3 \text{ ---- } R\text{-}PO_3H_2 \quad (IIa)$$

gebunden ist, mittels Reaktion der im organischen aliphatischen oder aromatischen Rest R enthaltenen chemisch reaktionsfähigen Gruppen mit den reaktionsfähigen Gruppen der Enzyme, die fixiert werden sollen, gegebenenfalls aktiviert durch geeignete Aktivatoren.

4. Verfahren nach Anspruch 3, dadurch gekennzeichnet, daß, wenn der unlösliche Komplex reaktionsfähige Carbonylgruppen, wie Aldehydgruppen, umfaßt, die durch das organische Phosphat eingebracht worden sind, diese reaktionsfähigen Gruppen direkt mit den Aminogruppen der Enzyme, die fixiert werden sollen, unter Bildung von Schiff'schen Basen reagieren.

5. Verfahren nach Anspruch 4, dadurch gekennzeichnet, daß die erhaltenen Schiff'schen Basen durch Reduktion mit einem geeigneten Reduktionsmittel, insbesondere Natriumcyanborhydrid, stabilisiert werden.

6. Verfahren nach Anspruch 3, dadurch gekennzeichnet, daß, wenn der unlösliche Komplex reaktionsfähige Aminogruppen enthält, diese durch einen geeigneten Aktivator, wie einem bifunktionellen Reaktionspartner der primären Amine aus der Gruppe umfassend vor allem Glutaraldehyd, Diisocyanate, Diisothiocyanate usw. . . ., aktiviert werden, um dazu fähig gemacht zu werden, mit den Aminogruppen der Enzyme, die fixiert werden sollen, zu reagieren.

7. Verfahren zum Katalysieren von Peptid-Synthesen, dadurch gekennzeichnet, daß man als enzymatischen Katalysator mindestens ein Enzym einsetzt, das durch Kombination mit einem Komplex Aluminiumoxid-organisches Phosphat unlöslich gemacht und durch Ausführung des Verfahrens nach einem der Ansprüche 3 bis 6 erhalten worden ist.

8. Verfahren nach Anspruch 7, dadurch gekennzeichnet, daß die Peptid-Synthesen bei einer Temperatur zwischen Raumtemperatur und etwa 45°C ausgeführt werden.

9. Verfahren nach einem der Ansprüche 7 oder 8, dadurch gekennzeichnet, daß das Reaktionsmedium frei ist von Phosphationen, deren Abwesenheit die Trennung des Enzyms von seinem Träger hervorrufen könnte.

10. Verfahren nach einem der Ansprüche 7 bis 9, dadurch gekennzeichnet, daß die unlöslich gemachten Enzyme die Synthese von Amidbindungen und Peptidbindungen katalysieren zwischen einem Aminosäurederivat, das eine blockierte Aminogruppe und eine freie oder veresterte Carboxylgruppe aufweist, und einem Aminosäurederivat, das eine freie Aminogruppe und eine blockierte Carboxylgruppe aufweist, die jeweils den nachfolgenden Formeln III und IV entsprechen

$$\begin{array}{l} Z - NH-CHCOOX \\ \qquad\quad\;\; | \\ \qquad\quad\; R_x \end{array} \qquad (III)$$

in der Z eine Amino-Schutzgruppe oder ein Peptid-Segment darstellt, X ein Wasserstoffatom oder eine Alkylgruppe bedeutet und $R_x$ die Seitenkette einer α-Aminosäure ist;

$$\begin{array}{l} H_2N - CH - COY \\ \qquad\quad\; | \\ \qquad\quad R_x \end{array} \qquad (IV)$$

in der Y eine Alkoxy- oder Alkoylaminogruppe oder ein Peptid-Segment bedeutet und $R_x$ die Seitenkette einer α-Aminosäure ist.

11. Anwendung des Komplexes nach Anspruch 1 als Träger für die Fixierung von Protein-Molekülen, insbesondere von Enzymen.

12. Anwendung des Komplexes nach Anspruch 1 als Mittel für die Isolierung und/oder Bestimmung von organischen Phosphaten, die in einem biologischen Medium enthalten sind, wie Mononucleotide, Phosphoglucide, Phospho-Proteine, Phospho-Aminosäuren, Pyridoxal-phosphat usw. . . ., durch Bindung dieser organischen Phosphate an Aluminiumoxid in wäßrigem Medium bei einem pH-Wert zwischen 2 und 8,5 und Freisetzung dieser organischen Phosphate aus dem entstandenen Komplex durch Einwirkung einer Lösung eines anorganischen Phosphates oder von Phosphorsäure, equilibriert auf einen pH-Wert im Bereich zwischen 2,0 und 8,5.

**Claims for the Contracting States BE, CH, DE, FR, GB, IT, LI, LU, NL, SE**

1. Novel solid complex which is characterized in that it is formed by alumina bonded to at least one phosphate funcitonal group of a bi- or polyfunctional compound containing at least one phosphate functional group and at least one chemically reactive group capable of forming a covalent bond with molecules of a proteinic nature and particularly enzymes.

2. Complex according to claim 1, characterized in that the bi- or polyfunctional compound bonded to the alumina is an organic phosphate which corresponds with one of formulae I or II below:

$$R - O - PO_3H_2 \qquad\qquad\qquad R - PO_3H_2$$
$$I \qquad\qquad\qquad\qquad\qquad II$$

in which R represents an aliphatic or aromatic organic group containing at least one chemically reactive group such as in particular, a primary amine, aldehyde or acid group.

3. Process for the preparation of the complex according to any one of claims 1 or 2, characterized in that it consists of fixing an organic phosphate which corresponds to formula I or II according to claim 1, to alumina, by contacting alumina and an organic phosphate for a sufficient time in an aqueous medium, substantially at ambient temperature, at a pH comprised between 2.0 and 8.5.

4. Process according to claim 3, characterized in that the medium in which the complexation is carried out is a medium with a high ionic strength.

5. Application of the complex according to any one of claims 1 or 2 as a support for the fixation of molecules of a proteinic nature, and particularly enzymes.

6. Application of the complex according to any one of claims 1 or 2 as a means for the isolation and/or assay of organic phosphates contained in a biological medium such as mononucleotides, phosphoglucides, phospho-proteins, phospho-amino-acids, pyridoxal-phosphate, etc. . . ., by coupling said organic phosphates with alumina, in an aqueous medium, at a pH comprised between 2 and 8.5, and release of these organic phosphates from the complex formed, by the action of an inorganic phosphate or phosphoric acid solution equilibrated to a pH comprised between 2.0 and 8.5.

7. Process for the preparation of insolubilized enzymes, characterized in that it consists of forming a complex body comprising an enzyme coupled by a covalent bond to a solid support of formula Ia or IIa below:

$$Al_2O_3 \text{ ---- } R\text{-}O\text{-}PO_3H_2 \quad \text{(Ia)} \qquad Al_2O_3 \text{ ---- } R\text{-}PO_3H_2 \quad \text{(IIa)}$$

by the reaction of chemically reactive groups which comprise the aliphatic or aromatic organic group R, with reactive groups of the enzymes to be fixed, possibly activated by suitable activators.

8. Process according to claim 7, characterized in that when the insoluble complex comprises carbonylic reactive groups such as aldehyde groups, introduced by the organic phosphate these reactive groups react directly with the amine functions of the enzymes to be fixed by forming Schiff bases.

9. Process according to claim 8, characterized in that the Schiff bases obtained are stabilized by reduction by means of a suitable reducing agent, particularly sodium cyanoborohydride.

10. Process according to claim 9, characterized in that when the insoluble complex comprises reactive groups constituted by amine groups, the latter are activated by a suitable activator such as a bifunctional reagent of primary amines taken from the group which comprises particularly glutaraldehyde, diisocyanates, diisothiocyanates, etc.... to be made suitable for reacting with the amine groups of the enzymes to be fixed.

11. By way of novel industrial products, enzymes insolubilized by association with a complex organic alumina-phosphate, obtained by employing the process according to any one of claims 7 to 10.

12. Process for the catalysis of peptide syntheses characterized by the employment as enzymatic catalyst of at least one enzyme insolubilized according to claim 11.

13. Process according to claim 12, characterized in that said peptide syntheses are performed at a temperature comprised between ambient temperature and 45°C.

14. Process according to any one of claims 12 or 13, characterized in that the reaction medium is free from phosphate ions whose presence could cause the separation of the enzyme and its support.

15. Process according to any one of claims 12 to 14, characterized in that the insolubilized enzymes catalyse the syntheses of amide and peptide linkages between an amino acid derivative having a blocked amine function and a free or esterified carboxylic function and an amino acid derivative having a free amine function and a blocked carboxylic function, which correspond respectively to the formulas III and IV below:

$$Z - NH-\underset{\displaystyle R_x}{\underset{|}{C}}HCOOX \qquad \text{(III)}$$

where Z represents a protective group of the amine function or a peptide segment
X represents a hydrogen atom or an alkyl group
$R_x$ represents a side chain of an α-amino acid;

$$H_2N - \underset{\displaystyle R_x}{\underset{|}{CH}} - COY \qquad \text{(IV)}$$

where Y represents an alkoxy or alkylamine group or again a peptide segment and $R_x$ represents the side chain of an α-amino acid.

**Claims for the Contracting State AT**

1. Process for the preparation of the solid complex formed by alumina bonded to at least one phosphate functional group of a bi- or polyfunctional compound containing at least one phosphate functional group and at least one chemically reactive group capable of forming a covalent bond with molecules of a proteinic nature and particularly enzymes, characterized in that it consists of fixing an organic phosphate which corresponds to formula I or II below:

$$R - O - PO_3H_2 \quad \text{(I)} \qquad R - PO_3H_2 \quad \text{(II)}$$

in which:
R represents an aliphatic or aromatic organic group comprising at least one chemically reactive group such as, in particular, a primary amine, aldehyde or acid group, on alumina, by contacting alumina and an organic phosphate, for a sufficient time, in an aqueous medium, substantially at ambient temperature, at a pH comprised between 2.0 and 8.5.

2. Process according to claim 1, characterized in that the medium in which the complexation is performed is a medium with a high ionic strength.

3. Process for the preparation of insolubilized enzymes, characterized in that it consists of forming a complex body comprising an enzyme coupled by covalent linkage to a solid support of formula Ia or IIa below:

$$Al_2O_3 \text{ --- } R\text{-}O\text{-}PO_3H_2 \quad (Ia) \qquad Al_2O_3 \text{ --- } R\text{-}PO_3H_2 \quad (IIa)$$

by the reaction of chemically reactive groups which comprise the aliphatic or aromatic organic group R with the reactive groups of the enzymes to be fixed, as the case may require, activated by suitable activators.

4. Process according to claim 3, characterized in that when the insoluble complex comprises carbonylic reactive groups such as aldehyde groups, introduced by the organic phosphate, these reactive groups react directly with the amine functions of the enzymes to be fixed, by forming Schiff bases.

5. Process according to claim 4, characterized in that the Schiff bases obtained are stabilized by reduction by means of a suitable reducing agent, particularly sodium cyanoborohydride.

6. Process according to claim 3, characterized in that when the insoluble complex comprises reactive groups constituted by amine groups, the latter are activated by a suitable activator such as a bifunctional reagent of the primary amines taken from the group which comprises particularly glutaraldehyde, diisocyanates, diisothiocyanates, etc. . . . to be made suitable for reacting with the amine groups of the enzymes to be fixed.

7. Process of catalysis of peptide syntheses characterized by the employment as an enzymatic catalyst of at least one enzyme insolubilized by association with an alumina-organic phosphate complex obtained by employing the process according to any one of claims 3 to 6.

8. Process according to claim 7, characterized in that said peptide syntheses are performed at a temperature comprised between ambient temperature and about 45°C.

9. Process according to any one of claims 7 or 8, characterized in that the reaction medium is free from phosphate ions whose presence could cause the separation of the enzyme and of its support.

10. Process according to any one of claims 7 to 9, characterized in that the insolubilized enzymes catalyse the syntheses of amide and peptide linkages between an amino acid derivative having a blocked amine function and a free or esterified carboxylic function and an amino acid derivative having a free amino function and a blocked carboxylic function which correspond respectively to formulae III and IV below:

$$Z - NH\text{-}\underset{\substack{|\\ R_x}}{CH}COOX \qquad (III)$$

where Z represents a protective group of the amine function or a peptide segment
X represents a hydrogen atom or an alkyl group
$R_x$ represents a side chain of an $\alpha$-amino acid:

$$H_2N - \underset{\substack{|\\ R_x}}{CH} - COY \qquad (IV)$$

where Y represents an alkoxy or alkylamine group, or again a peptide segment and
$R_x$ represents the side chain of an $\alpha$-amino acid.

11. Application of the complex according to claim 1, as a support for the fixation of molecules of a proteinic nature and particularly enzymes.

12. Application of the complex according to claim 1, as a means for the isolation and/or the assay of organic phosphates contained in a biological medium such as mononucleotide, phophosglucids, phosphoproteins, phospho-amino-acids, pyridoxal-phosphate, etc.... by coupling said organic phosphates with alumina, in an aqueous medium, at a pH comprised between 2 and 8.5, and liberation of these organic phosphate from the complex formed, by the action of a solution of inorganic phosphate or of phosphoric acid equilibrated to a pH comprised between 2.0 and 8.5.